# EUROPÄISCHE PATENTSCHRIFT

(11) **EP 3 943 029 B1**
(45) Veröffentlichungstag und Bekanntmachung des Hinweises auf die Patenterteilung: **02.04.2025**
(21) Anmeldenummer: 20187417.9
(22) Anmeldetag: 23.07.2020
(51) Int. Cl.: A61B 18/04, A61B 18/00, A61B 18/12

(54) **PLASMASONDE MIT VERBESSERTEM ZÜNDVERHALTEN**
PLASMA PROBE WITH IMPROVED IGNITION BEHAVIOUR
SONDE À PLASMA À COMPORTEMENT D'ALLUMAGE AMÉLIORÉ

(43) Veröffentlichungstag der Anmeldung: 26.01.2022
(73) Patentinhaber: Erbe Elektromedizin GmbH, 72072 Tübingen (DE)
(72) Erfinder: Fischer, Klaus, 72202 Nagold (DE)
(74) Vertreter: Rüger Abel Patentanwälte PartGmbB

(56) Entgegenhaltungen:
- DE-T2- 69 636 399
- US-A1- 2018 353 233
- US-A1- 2020 188 014

## Beschreibung

Die Erfindung betrifft eine Plasmasonde, insbesondere zur Plasmakoagulation von biologischem Gewebe. Weiter insbesondere betrifft die Erfindung eine Plasmasonde zum endoskopischen Einsatz.

Aus der DE 69636399 T2 ist eine Plasmasonde bekannt, die aus einem Rohr aus biegsamen Material besteht. Dieses Rohr wird durch einen Arbeitskanal eines Endoskops in den Körper eines Patienten geführt. Das Rohr weist ein Endstück auf, das aus einem temperaturbeständigen Material wie PTFE oder Keramik gefertigt ist. Ebenso kann das Rohr aus PTFE (Polytetrafluoräthylen) bestehen. In dem von dem Rohr und dem Endstück umschlossenen Kanal ist eine Elektrode angeordnet, deren Spitze aus der Öffnung des Rohrs bzw. des Endstücks nicht herausragt. Die Elektrode ist an eine Spannungsquelle angeschlossen. Der von dem Rohr umschlossene Kanal wird von Inertgas, beispielsweise Argon, durchströmt, sodass von der Sonde ein Plasmastrahl ausgeht. Eine Regulationseinheit gestattet dabei die Einstellung des Volumenstroms des Gases, die zumindest zwei verschieden große Gasströme bereitstellen kann. Im Standby-Betrieb ist der Gasstrom gering. Um sicherzustellen, dass in die Sonde eingedrungene Fluide bei Plasmazündung aus der Sonde wieder ausgetrieben sind, wird der Gasstrom während des Betriebs erhöht. Dadurch kann vermieden werden, dass sich in dem Rohr Blutreste ansammeln, was bei einer späteren Operation an einem anderen Patienten die Gefahr einer Infektion hervorrufen würde (siehe Abschnitt [0040]).

Aus der DE 100 30 111 B4 ist eine weitere Plasmasonde bekannt, die aus einem dünnen Schlauch mit einem distalen hitzebeständigen Endstück und einer in dem Lumen des Schlauchs angeordnete Elektrode besteht. Die Elektrode wird insbesondere durch ein mit einer distalen Spitze versehenes Blech gebildet, dessen Längskanten sich an der Innenseite des Schlauchs abstützen.

Aus der EP 0 957 793 B2 ist eine Plasmasonde mit einem Schlauch aus PTFE oder einem ähnlichen Material bekannt. Dieser Schlauch ist am distalen Ende mit einem Endstück versehen, das aus PTFE oder aus Keramik besteht. In dem Schlauch ist eine Elektrode angeordnet, die von dem von dem Schlauch herangeführten Edelgas umströmt wird und einen Plasmastrahl bildet. Wiederum kann der Gasstrom in Standby-Phasen verringert und bei Aktivierung erhöht werden, um ein Eindringen von Körperflüssigkeiten und Verschleppung derselben durch die Sonde auf andere Patienten zu vermeiden.

Weiter wird in der EP 0 957 793 B2 eine Plasmasonde vorgeschlagen, deren Endstück aus Keramik besteht. Unmittelbar an das proximale Ende des hohlen Schafts des keramischen Endstücks ist eine rohrförmige Elektrode angeschlossen, deren Innendurchmesser mit dem Innendurchmesser des keramischen Endstücks übereinstimmt. Diese Anordnung dient zur Koagulation großer Gewebeflächen.

Aus der US 2018/0353233 A1 ist eine Argon-PlasmaSonde mit einem Schlauchkörper bekannt, in dessen Zentrum eine Elektrode angeordnet ist. An dem distalen Ende der Sonde sind hydrophobe Wandbereiche vorgesehen, um die Ansammlung von Flüssigkeit zu minimieren.

Im praktischen Betrieb zeigen die vorbeschriebenen Sonden gelegentlich schlechtes Zündverhalten. Dies bedeutet, dass zum Aufbau einer Plasmaentladung entweder besonders hohe Spannung genutzt werden müssen oder die Sonde sehr nahe an das zu behandelnde Gewebe herangeführt werden muss.

Davon ausgehend ist es Aufgabe der Erfindung, eine Plasmasonde mit verbessertem Zündverhalten anzugeben.

Diese Aufgabe wird mit der Plasmasonde nach Anspruch 1 gelöst:
Die erfindungsgemäße Plasmasonde weist eine flexible Fluidleitung auf, die beispielsweise durch einen dünnen Schlauch aus einem geeigneten Kunststoff, wie beispielsweise PTFE oder einem anderen geeigneten Kunststoff, bestehen kann. Die Fluidleitung begrenzt wenigstens einen Kanal, dessen proximales Ende mit einer Gasquelle verbindbar ist. Die Gasquelle dient dazu, den Kanal mit einem definierten Gasstrom zu versorgen, der an einer an dem distalen Ende der Fluidleitung ausgebildeten Ausströmöffnung austritt.

Weiter weist die Plasmasonde eine Elektrode auf, die über eine elektrische Leitung an eine elektrische Quelle anschließbar ist. Die Leitung kann ein sich durch den Kanal erstreckender Draht, eine Litze oder dergleichen oder ein an oder in der Wandung des Schlauchs geführter Leiter sein. Die elektrische Quelle kann ein Hochfrequenzgenerator sein, wie er in medizinischen Geräten zur Versorgung solcher Sonden vorhanden ist. Die Elektrode ist vorzugsweise in Nachbarschaft zu der Ausströmöffnung angeordnet. Das vorzugsweise spitz ausgebildete Ende der Elektrode kann in dem Kanal vorzugsweise so angeordnet sein, dass es nicht aus der Ausströmöffnung herausragt. Damit ist eine Verletzung von biologischem Gewebe durch mechanische Einwirkung der Elektrode ausgeschlossen. Alternativ kann sich die Elektrode durch die Ausströmöffnung hindurch erstrecken und endseitig einen stumpfen, vorzugsweise elektrisch isolierenden, Körper tragen, um wiederum Verletzungen von Gewebe zu vermeiden. Eine solche Sonde eignet sich für die omnidirektionale laterale Plasmaabgabe.

Erfindungsgemäß ist an der Elektrode ein hydrophober Oberflächenbereich ausgebildet. Vorzugsweise betrifft dies insbesondere einen dem distalen Ende der Elektrode nahen oder an das distale Ende der Elektrode angrenzenden Abschnitt der Elektrode. Die Oberfläche der Elektrode kann auch insgesamt hydrophob ausgebildet sein.

Durch die hydrophobe Ausbildung der Oberfläche der Elektrode wird deren Zündverhalten wesentlich verbessert. Wenn beim Einführen der Plasmasonde in das Endoskop Flüssigkeiten, beispielsweise Spülflüssigkeit, die Plasmasonde benetzen, neigen solche Flüssigkeiten auch zum Eindringen in die offene Ausströmöffnung der Plasmasonde. Solche Flüssigkeitströpfchen können den Gasstrom beeinträchtigen, Turbulenzen und Asymmetrien in der Gasströmung erzeugen und die Elektronenemission an der Spitze der Elektrode behindern. Ist die Elektrode hingegen wasserabweisend (hydrophob), werden solche Effekte reduziert oder vermieden.

Der hydrophobe Oberflächenbereich der Elektrode verhindert auch, dass sich während des Betriebs der Plasmasonde biologisches Gewebe, Gewebeflüssigkeit oder aus dem biologischen Gewebe herrührende Produkte an der Elektrode anlagern und deren Oberflächenleitfähigkeit verändern. Damit wird auch ein Wandern des Entladungsfußpunkts und insbesondere ein Wandern des Entladungsfußpunkts von der Elektrodenspitze weg in den Kanal der Sonde hinein unterbunden. So wird auch die sonst drohende thermische Überbelastung der Sonde verhindert. Eine solche thermische Überbeanspruchung könnte sonst auch zur Verformung des Fluidleiters führen, was wiederum die Gasströmung und die Ausbildung des Plasmastroms massiv behindern würde. Auch könnte bei entsprechender Sondenschädigung ein direkter Kontakt zwischen Elektrode und Gewebe erfolgen. Diese Folgewirkungen eines wandernden Entladungsfußpunkts vermeidet die Erfindung weitgehend oder ganz.

Die wasserabweisende Ausbildung der Elektrode, insbesondere des spitzennahen Oberflächenbereichs derselben, führt außerdem zu erhöhter Zündwilligkeit, insbesondere bei widrigen, d.h., besonders nassen Einsatzbedingungen.

Die erfindungsgemäße Plasmasonde kann eine Fluidleitung aufweisen, die selbst aus wärmebeständigem Material besteht oder an ihrem distalen Ende mit einem wärmebeständigen Endstück versehen ist, durch das sich der Kanal erstreckt. Das Endstück kann einen Innendurchmesser aufweisen, der mit dem Innendurchmesser des Schlauchs übereinstimmt oder auch geringfügig kleiner ist als dieser. Vorzugsweise erstreckt sich die Elektrode wenigstens teilweise in das Endstück, sodass der Entladungsfußpunkt kurz vor der Ausströmöffnung innerhalb des Endstücks angeordnet ist.

Zusätzlich zu dem an der Elektrode ausgebildeten hydrophoben Oberflächenbereich können weitere hydrophobe Oberflächenbereiche vorgesehen sein. Dadurch können Kapillareffekte verhindert werden. Das heißt es wird einerseits ein leichteres Abblasen von Flüssigkeit ermöglicht und es könnte durch die Verminderung von Kapillarkräften die Flüssigkeitsaufnahme reduziert werden. Dazu können das Endstück und/oder der Schlauch insbesondere innen, an den Kanal angrenzend, bedarfsweise jedoch auch um die Ausströmöffnung herum und/oder an der Außenseite des Endstücks und/oder des Schlauchs hydrophobe Oberflächenbereiche aufweisen. Mit diesen Maßnahmen lassen sich Zündwilligkeit und Standfestigkeit der Sonde in besonderer Weise fördern.

Darüber hinaus ist es möglich, die Elektrode hohl auszubilden, sodass die Elektrode einen von Gas durchströmbaren Kanal aufweist. Diese Elektrode kann insbesondere in der Nähe ihres distalen Endes oder auch insgesamt an ihrer Außenseite mit einer hydrophoben Oberfläche versehen sein. Es ist auch möglich, den von der Elektrode umschlossenen Kanal mit einer hydrophoben Oberfläche zu versehen.

Weiter ist es möglich, zur Lagerung der Elektrode in dem Kanal und/oder zur Beeinflussung der Gasströmung ein Strömungsleitelement aus Metall oder einem elektrischen Isolator, wie Kunststoff oder Keramik vorzusehen, welches Strömungsleitelement dazu eingerichtet sein kann, den Gasstrom im Zentrum des Querschnitts, d.h., in Nachbarschaft der Elektrode im Vergleich zu Randbereichen des Querschnitts zu drosseln. Auch diese Maßnahme dient der Erhöhung der Zündwilligkeit. Auch das Strömungsleitelement kann mit einer hydrophoben Oberfläche ausgestattet und dazu z.B. mit einer hydrophoben Oberflächenstruktur oder Beschichtung versehen sein.

Um die gewünschten Oberflächenabschnitte hydrophob zu gestalten, können diese mit einer hydrophoben Beschichtung versehen sein, beispielsweise Silikonöl, Fluoropolymere, wie z.B. Polytetrafluoräthylen, Parylene, PFA (Perfluoroalkoxy), Polyamid, Polyethylen, einem SilikonElastomer oder dergleichen. Hydrophobe Beschichtungen können z.B. durch einen PVD oder CVD-Prozess aufgebracht werden. Zusätzlich oder alternativ ist es möglich, die wasserabweisende Wirkung der betreffenden Oberflächenbereiche durch Mikrostrukturierung der Oberflächen zu erzielen. Dazu können die betreffenden Oberflächenbereiche mit einer Vielzahl sich kreuzender feinster Nuten versehen sein, die ein Raster winziger Vorsprünge freilassen. Diese Vorsprünge halten Flüssigkeitstropfen mit ihren Stirnflächen von den Nutböden fern, sodass Flüssigkeit leicht abperlen kann.

Weitere Einzelheiten vorteilhafter Ausführungsformen der Erfindung sind Gegenstand von Unteransprüchen, sowie der Zeichnung oder der Beschreibung. Es zeigen:
Figur 1 ein medizinisches Gerät mit einer daran angeschlossenen Plasmasonde, in schematisierter Darstellung,
Figur 2 die Plasmasonde nach Figur 1, in längsgeschnittener Darstellung ihres distalen Endabschnitts,
Figur 3A - 3D die Elektrode der Plasmasonde nach Figur 2, in längsgeschnittener vergrößerter Darstellung ihres distalen Endes, in unterschiedlichen Ausführungsformen,
Figur 4 eine abgewandelte Ausführungsform der erfindungsgemäßen Plasmasonde, in Längsschnittdarstellung ihres distalen Endes,
Figur 5 eine weitere Ausführungsform einer erfindungsgemäßen Plasmasonde, in längsgeschnittener Darstellung ihres distalen Endes,
Figur 6 die Plasmasonde nach Figur 5 mit Blickrichtung auf das distale Ende,
Figur 7 und 8 weitere Ausführungsformen erfindungsgemäßer Plasmasonden, jeweils in Längsschnittdarstellung ihres distalen Endes,
Figur 9 die Plasmasonde nach Figur 8 unter Veranschaulichung des Strömungsprofils,
Figur 10 eine Ausführungsform der erfindungsgemäßen Plasmasonde, in längsgeschnittener Darstellung ihres distalen Endes.

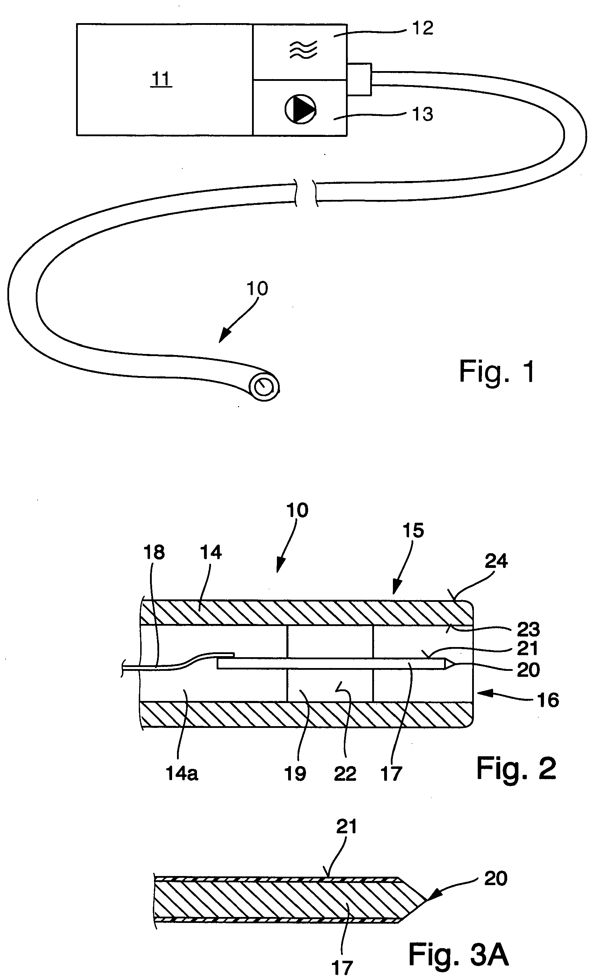

In Figur 1 ist eine Plasmasonde 10 veranschaulicht, wie sie durch ein nicht weiter veranschaulichtes Endoskop zur Durchführung einer Plasmabehandlung an einem Patienten Anwendung finden kann. Die Plasmasonde 10 ist dazu an ein speisendes Gerät 11 oder eine Geräteanordnung angeschlossen, das oder die zur Versorgung der Plasmasonde 10 mit elektrischem Strom und einem Inertgas dient. Das Gerät weist dazu einen elektrischen Generator, beispielsweise einen HF-Generator 12 auf, der dazu geeignet ist, eine hochfrequente Wechselspannung abzugeben, die ausreicht, um eine Plasmaentladung zu zünden und aufrecht zu erhalten.

Zur Versorgung mit Inertgas dient eine Gasquelle 13, die ein geeignetes Gas, beispielsweise Argon, liefert. Die Gasquelle 13 ist dabei darauf eingerichtet, Gas dosiert und kontrolliert zu liefern, um die Plasmasonde 10 mit einem geeigneten Volumenstrom Gas zu versorgen.

Die Plasmasonde 10 ist an ihrem proximalen Ende, beispielsweise über einen geeigneten Stecker, an den HF-Generator 12 und an die Gasquelle 13 angeschlossen.

Die Plasmasonde 10 besteht im Wesentlichen aus einer flexiblen Fluidleitung 14 beispielsweise in Gestalt eines Kunststoffschlauchs. Dieser kann aus PTFE oder einem anderen für medizinische Anwendungen geeigneten Kunststoff bestehen. Die Fluidleitung 14 umschließt einen Kanal 14a, der zu der an dem distalen Ende 15 des Schlauchs 14 vorgesehenen Ausströmöffnung 16 führt. In dem distalen Ende 15 ist eine Elektrode 17 beispielsweise in Form eines endseitig zugespitzten metallischen Stifts oder eines Drahts angeordnet. Während das distale Ende der Elektrode 17 in dem Kanal 14a vorzugsweise etwa mittig so angeordnet ist, dass es nicht aus der Ausströmöffnung 16 herausragt, ist das proximale Ende der Elektrode 17 mit einer geeigneten elektrischen Leitung 18 verbunden, die zu dem Generator 12 führt.

Zur Positionierung der Elektrode 17 in den Kanal 14a kann eine geeignete Halteeinrichtung vorgesehen sein, beispielsweise ein diametral in dem Kanal 14a positionierter plattenförmiger Halter 19, der sich mit beiden Längskanten an der Innenwandung des Schlauchs 14 abstützt. Die Elektrode 17 kann mit dem Halter 19 verklemmt, verschweißt oder anderweitig verbunden sein. Es ist auch möglich, die Elektrode 17 und den Halter 18 aus ein einziges Teil, beispielsweise ein rhombisches Blechteil auszubilden, dessen Spitze die Position der Spitze 20 der Elektrode 17 einnimmt und dessen Längskanten sich innen an dem Schlauch 14 abstützen.

Erfindungsgemäß weist die Elektrode 17 mindestens einen hydrophoben Oberflächenbereich 21 auf. Dieser umgibt vorzugsweise wenigstens das distale Ende der Elektrode 17. Insbesondere kann der hydrophoben Flächenbereich 21 die zylindrische Außenumfangsfläche der Elektrode 17 einnehmen. Figur 3A veranschaulicht den hydrophoben Flächenbereich 21, der durch eine wasserabweisende Beschichtung der Elektrode 17 erzeugt sein kann. Die beispielsweise aus Wolfram oder einem anderen Metall bestehende Elektrode 17 ist dazu an ihrer Außenumfangsfläche mit einer Schicht aus einem wasserabweisenden Material beispielsweise PTFE, Silikonöl oder dergleichen versehen. Vorzugsweise ist die Spitze 20 oder ein Abschnitt derselben jedoch freigelassen, also unbeschichtet, wobei unter dem Begriff der "Spitze" der gesamte sich verjüngende, z.B. kegelförmige Bereich der Elektrode 17 gemeint ist.

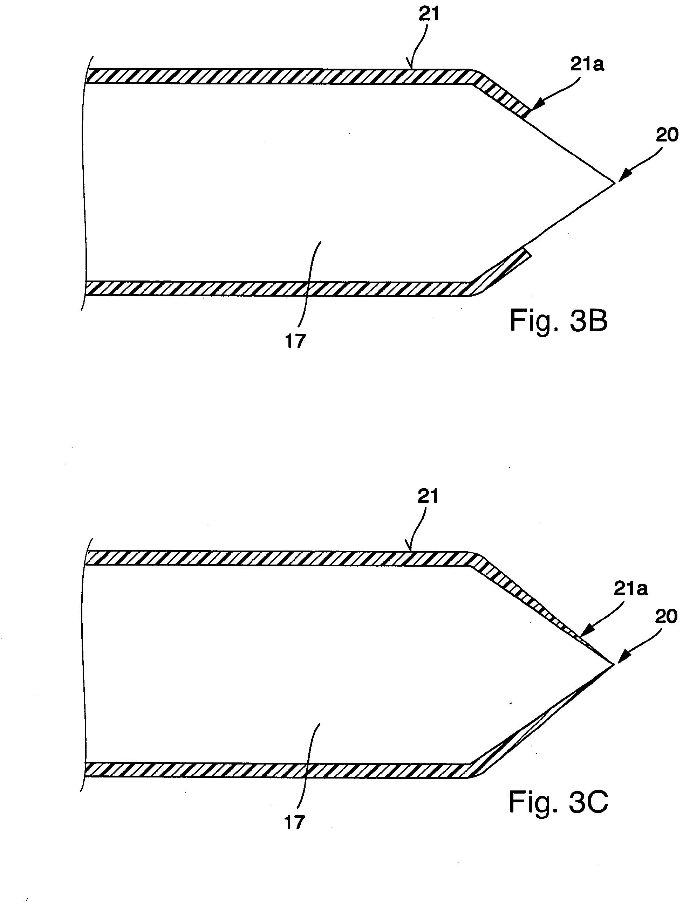

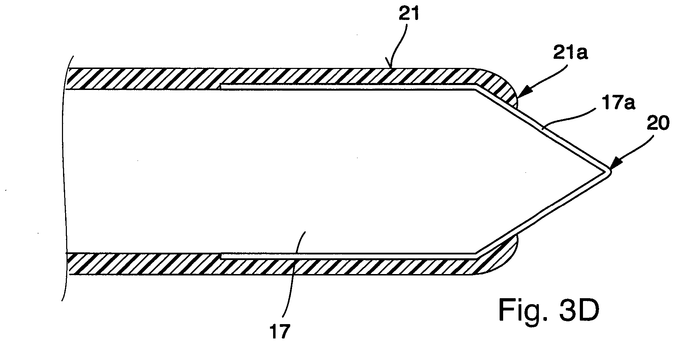

Die Elektrode 17 kann auch gemäß Figur 3B bis in den Bereich der Spitze hinein mit einer Beschichtung versehen sein, die den hydrophoben Flächenbereich 21 bildet. Die Beschichtung kann sich Dabei mit unverminderter Dicke bis zu ihrem Ende 21a erstrecken, oder, wie es Figur 3C veranschaulicht, zu ihrem Ende 21a hin in ihrer Dicke kontinuierlich oder in Stufen abnehmen. Außerdem kann jede der vorbeschriebenen Elektroden 17, wie es Figur 3D am Beispiel der Elektrode nach Figur 3B darstellt, eine elektrisch gut leitende Schicht 17a tragen, wie z.B. eine Schicht aus Silber. Diese wird von der Beschichtung des hydrophoben Flächenbereichs 21 zu der oder bis in den Bereich der Spitze 20 bedeckt. Die Elektrode 17 selbst kann bei allen Ausführungsbeispielen aus Edelstahl, Stahl, Wolfram, Hartmetall oder einem anderen hochschmelzenden Material bestehen.

Die wasserabweisende Wirkung kann sich in dem hydrophoben Flächenbereich 21 durch die geringe Oberflächenenergie des verwendeten Materials ergeben, die vorzugsweise geringer als 20 mJ/m² ist. Zusätzlich oder alternativ kann die hydrophobe Wirkung in dem Flächenbereich 21 durch eine Mikrostrukturierung der Oberfläche erreicht werden. Dazu wird der Oberflächenbereich 21 so geformt, dass von ihm eine Vielzahl sehr kleiner stachelartiger Forstätze radial vorstehen. Beispielsweise wird dies erreicht, indem der Oberflächenbereich 21 zunächst beispielsweise mittels Lasers mit einer Schraubennut und nachfolgend mit axialen Nuten versehen wird. Die Länge der verbleibenden Vorsprünge und deren Abstände zueinander sind dabei so aufeinander abgestimmt, dass ein auf den Spitzen dieser Vorsprünge liegender Wassertropfen mit dem sich zwischen den Vorsprüngen ausbildenden Meniskus den Nutboden aufgrund seiner eigenen Oberflächenspannung nicht berührt. Es sind auch andere Verfahren zur Mikrostrukturierung oder Beeinflussung der Oberflächenrauheit anwendbar, wie z.B. Trockenätzen oder das Aufbringen von Nanopartikeln.

Die Spitze 20 kann sich infolge ihrer großen Krümmung ihrer Kegelfläche und deren Spitze als hydrophob erweisen.

Des Weiteren kann der Halter 19 einen hydrophoben Oberflächenbereich 22 aufweisen, der die Oberfläche des Halters 19 ganz oder teilweise einnimmt. Wiederum kann der hydrophobe Oberflächenbereich 22 durch geeignete Mikrostrukturierung der Oberfläche oder durch Beschichtung derselben mit wasserabweisendem Material erreicht werden. Zu der Strukturierung der Oberfläche oder den Beschichtungsmaterialien gilt die obige zu der Elektrode 17 und dem hydrophoben Oberflächenbereich 21 gegebenen Beschreibung entsprechend.

Zudem kann der Schlauch 14 innen und/oder außen hydrophob sein und entsprechende hydrophobe Oberflächenbereiche 23, 24 aufweisen. Ebenso kann seine Stirnseite hydrophob sein. Erreicht werden kann dies durch geeignete Materialwahl, beispielsweise indem der Schlauch 14 aus PTFE besteht. Die hydrophobe Wirkung seiner Oberfläche kann durch Mikrostrukturierung und oder Beschichtung oder Materialwahl wie vorstehend erläutert erhöht werden.

Die insoweit beschriebene Plasmasonde 10 arbeitet wie folgt:
In Betrieb strömt ein Gasstrom, beispielsweise ein Argon-Strom definierter Größe durch den Kanal 14a. Die Elektrode 17 ist z.B. mit hochfrequenter Wechselspannung beaufschlagt. Der Patient ist an eine Neutralelektrode angeschlossen. Ausgehend von der Spitze 20 bildet sich nun ein Plasmastrom, der auf das Gewebe trifft.

Bei Betriebsunterbrechung oder vor Betriebsbeginn kann Flüssigkeit, beispielsweise Wasser, Körperflüssigkeit, Spülflüssigkeit oder dergleichen an die Ausströmöffnung 16 gelangen. Jedoch vermeiden die hydrophoben Oberflächenbereiche 21 sowie auch 22, 23, 24 das Eindringen der Flüssigkeiten in den Kanal 14a oder wenigstens das Festsetzen von Flüssigkeitstropfen in dem Kanal 14a und an der Elektrode 17. Schon ein sehr geringer Gasstrom reicht somit aus, die Elektrode 17 und den Kanal 14a von eingedrungener Flüssigkeit zu befreien. Die insoweit trocken gebliebene Elektrode 17 kann mit großem Abstand zum Gewebe leicht zünden. Ein dabei gering gehaltener Gasstrom unterstützt die Zündwilligkeit.

Gleiches gilt beim Einführen der Plasmasonde 10 durch ein Endoskop in den Patienten vor Behandlungsbeginn. Auch in diesem Fall kann Flüssigkeit in den Kanal 14a vordringen, die dann schon mit sehr geringen Gasflüssen von der Elektrode 17 fortgespült werden kann.

An der insoweit grundsätzlich erläuterten Erfindung sind zahlreiche Abwandlungen möglich:

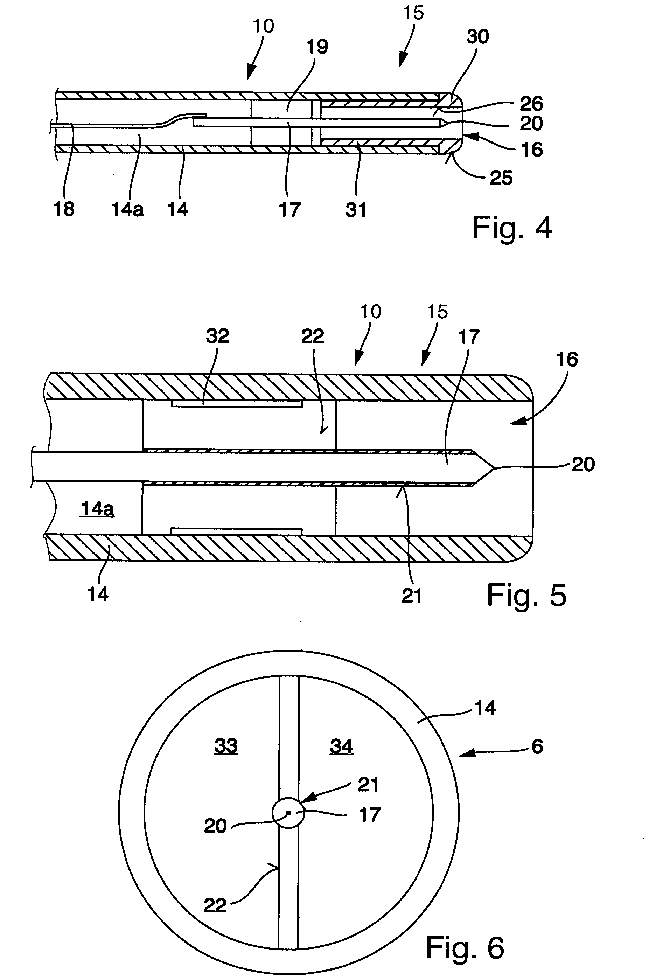

Figur 4 veranschaulicht eine abgewandelte Plasmasonde 10, die zusätzlich zu der Plasmasonde 10 nach Figur 2 in dem distalen Ende 15 des Schlauchs 14 einen keramischen Einsatz 30 aufweist. Dieser weist einen hohl ausgebildeten länglichen Schaft 31 auf, der in den Schlauch 14 eingeschoben ist. Der Kanal 14a erstreckt sich durch den Schlauch 14 und den Schaft 31 bis zu der Ausströmöffnung 16. Die Spitze 20 der Elektrode 17 ist in dem Einsatz 30 vorzugsweise in der Nähe der Ausströmöffnung 16 angeordnet. Der Schaft 31 dient der Wärmeverteilung und Ableitung. Der Einsatz 30 kann im Bereich seines distalen Endes außen mit einem hydrophoben Oberflächenbereich 25 versehen sein. Gleichfalls kann er innen an seiner Wandung einen hydrophoben Oberflächenbereich 26 aufweisen. Die wasserabweisende Wirkung der Oberflächenbereiche 25, 26 kann auf jede der vorbeschriebenen Weisen durch geeignete Stoffwahl und/oder Strukturierung der Oberfläche erreicht werden. Ansonsten gilt die im Zusammenhang mit den Figuren 1 bis 2 gegebene Beschreibung entsprechend.

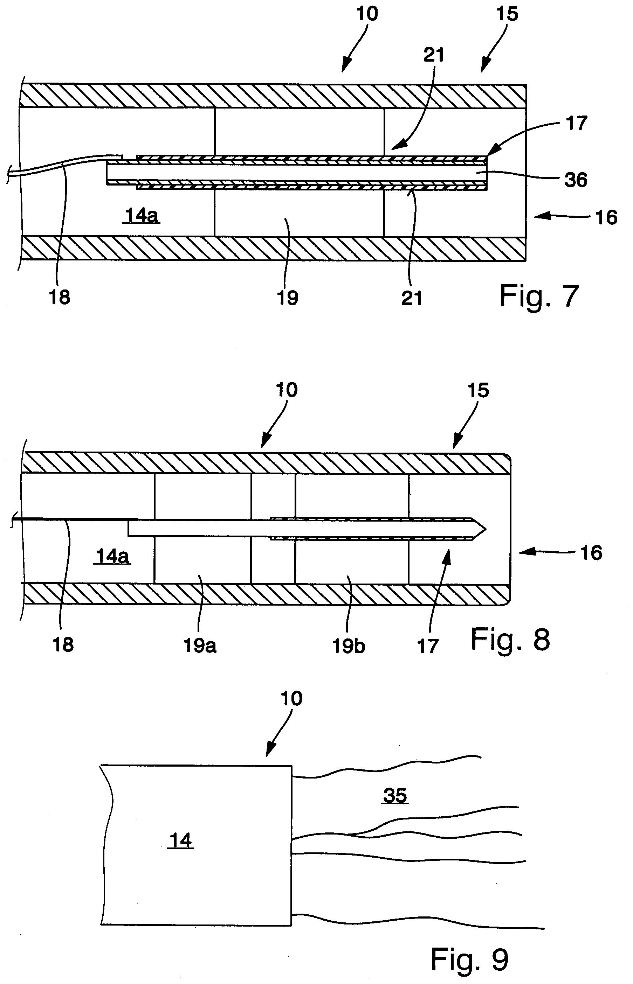

Die Plasmasonde 10 nach Figur 2 ist in den Figuren 5 und 6 nochmals gesondert dargestellt. Der Halter 19 kann als Kühlelement ausgebildet sein und an seinen schlauchseitigen Rändern eine oder mehrere Ausnehmungen aufweisen, die die Berührungsfläche zu dem Schlauch minimieren. Der Halter 19 kann aus einem Metall, einem Keramikmaterial oder einem Kunststoff oder einem Elastomer, wie z.B. Silicon, gefertigt sein. Zu beiden Seiten des Halters 19 sind, wie Figur 6 zeigt, Strömungskanäle 33, 34 vorgesehen, durch die Gas, z.B. Argon zu dem offenen Schlauchende hin strömen kann. Figur 9 veranschaulicht die austretende Gasströmung 35 und das sich darin ausbildende Plasma.

Wiederum kann, wie Figur 5 zeigt, die Elektrode 17 einen hydrophoben Oberflächenbereich 21 aufweisen, der sich vorzugsweise von der Spitze 20 bis zum den Halter 19 erstreckt. Auch kann der Halter 19 insbesondere an seiner Stirnseite und/oder in den Strömungskanälen 33, 34 hydrophobe Oberflächenbereiche 27, 28 aufweisen. Die Oberflächenbereiche 27, 28 können hydrophob sein, weil der Halter 19 selbst aus hydrophobem Material besteht, weil es mit hydrophobem Material beschichtet ist und/oder weil seine Oberfläche im Sinne eines Lotuseffekts mikrostrukturiert ist. Zur Mikrostrukturierung gilt die im Zusammenhang mit der Elektrode 17 gegebene Erläuterung entsprechend. Im Übrigen gilt auch die vorige Beschreibung der Figuren 1 bis 4 für die Ausführungsform nach Figuren 5 und 6 ergänzend entsprechen.

Eine weiter abgewandelte Ausführungsform der Plasmasonde 10 ist in Figur 7 veranschaulicht. Für diese Ausführungsform gilt zunächst die Beschreibung der Plasmasonde 10 nach Figur 5 und 6. Anders als dort ist die Elektrode 17 jedoch hohl und somit ohne Spitze ausgebildet. Sie umgrenzt einen engen Kanal 36. Die somit als Röhrchen ausgebildete Elektrode 17 ist wiederum mit dem Draht 18 oder sonstigen Leiter verbunden. Ihr distales Ende durchragt die Ausströmöffnung 16 nicht. An ihrer Außenseite trägt die Elektrode 17 z.B. wiederum die hydrophobe Beschichtung. Der Oberflächenbereich 21 kann hydrophob sein, weil die Elektrode 17 selbst aus hydrophobem Material besteht, weil sie mit hydrophobem Material beschichtet ist und/oder weil ihre Oberfläche im Sinne eines Lotuseffekts mikrostrukturiert ist.

Eine weitere Abwandlung der erfindungsgemäßen Plasmasonde 10 ergibt sich aus Figur 8. Die dort dargestellte Plasmasonde 10 weist zur Lagerung der Elektrode 17 zwei Halter 19a, 19b auf, die das Lumen des Schlauchs durchqueren und gegeneinander um die Elektrode 17 verdreht angeordnet sein können. Jedes der genannten Elemente, insbesondere die Elektrode 17 sowie einer der Halter 19a, 19b oder auch beide, können eine hydrophobe Oberfläche aufweisen und dazu aus hydrophoben Material bestehen oder durch Beschichtung mit hydrophoben Material oder Oberflächenstrukturierung hydrophob ausgestaltet sein.

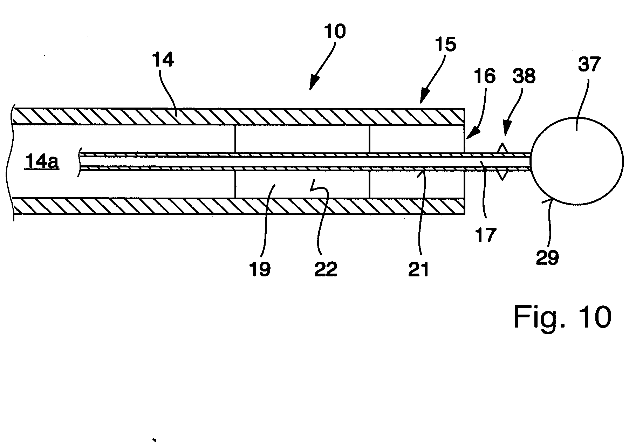

Die Elektrode 17 muss nicht zwingend innerhalb des Kanals 15 hinter der Ausströmöffnung 16 zurückstehen. Es ist, wie sich an der Plasmasonde 10 nach Figur 10 erkennen lässt, auch möglich, die Elektrode 17 aus der Ausströmöffnung 16 herauszuführen. In diesem Fall ist die Elektrode 17 vorzugsweise mit einem stumpfen, vorzugsweise isolierenden Körper 37, beispielsweise einer Keramikkugel, versehen, die einen direkten galvanischen Kontakt zwischen der Elektrode 17 und umgebenden biologischem Gewebe ebenso verhindert, wie eine mechanische Schädigung des Gewebes durch die Elektrode 17. Die Elektrode 17 kann von einem Halter 19, wie es in Figur 10 dargestellt ist, oder auch von mehreren Haltern ähnlich Figur 8 getragen sein. Als Entladungsfußpunkt kann die Elektrode 17 vor der Ausströmöffnung 16 angeordnete, sich radial von der Elektrode 17 weg erstreckende Spitze 38 aufweisen. Die Elektrode 17 kann wiederum den hydrophoben Oberflächenbereich 21 aufweisen. Ein weiterer hydrophober Oberflächenbereich 29 kann an dem Isolator 37 ausgebildet sein und diesen ganz oder teilweise einnehmen. Der Oberflächenbereich 29 kann hydrophob sein, weil der Isolator 37 selbst aus hydrophobem Material besteht, weil er mit hydrophobem Material beschichtet ist und/oder weil seine Oberfläche im Sinne eines Lotuseffekts mikrostrukturiert ist. Ansonsten gelten die vorigen Beschreibungen, insbesondere hinsichtlich der Ausbildung hydrophober Oberflächenbereiche 21 bis 27 entsprechend.

Bei allen Ausführungsformen gemäß Figur 5 bis 10 kann in das distale Ende 15 des Schlauchs 14 ein hohlzylindrischer Einsatz zum thermischen Schutz des Schlauchs 14 eingesetzt sein. Der Innendurchmesser dieses Einsatzes kann dem des Kanals 14a entsprechen oder auch geringfügig geringer sein als dieser. der Einsatz kann insbesondere an seiner inneren Wandung und oder an wenigstens einer seiner Stirnseiten hydrophob sein. Dazu kann er selbst aus hydrophobem Material bestehen, mit hydrophobem Material beschichtet und/oder an der Oberfläche im Sinne eines Lotuseffekts mikrostrukturiert sein.

Eine erfindungsgemäße Plasmasonde 10 weist eine Fluidleitung 14, die vorzugsweise als Schlauch ausgebildet ist, und eine darin angeordnete Elektrode 17 auf, deren Spitze 20 in der Nähe der Ausströmöffnung 16 steht. Die Elektrode 17 weist einen hydrophob ausgebildeten Oberflächenbereich 21 auf. Optional können weitere Elemente der Plasmasonde 10, insbesondere in Nachbarschaft zu der Ausströmöffnung 16, mit hydrophoben Oberflächen ausgestattet sein. Eine solche Sonde weist eine deutlich verbesserte Zündwilligkeit und aufgrund der Konstanz des sich ergebenden Entladungsfußpunkts eine verbesserte Standfestigkeit auf.

### Bezugszeichen:

- 10: Plasmasonde
- 11: Gerät
- 12: HF-Generator
- 13: Gasquelle
- 14: Fluidleitung (Kunststoffschlauch)
- 15: distales Ende der Fluidleitung
- 16: Ausströmöffnung
- 17: Elektrode
- 18: Leitung
- 19: Halter
- 20: Spitze der Elektrode 17
- 21 - 29: hydrophobe Oberflächenbereiche
- 21a: Ende des hydrophoben Oberflächenbereichs
- 30: Einsatz
- 31: Schaft
- 32: Ausnehmung
- 33: Strömungskanal
- 34a: Strömungskanal
- 35: Gasströmung
- 36: Kanal
- 37: Isolator
- 38: Spitzen

## Patentansprüche

1. Plasmasonde (10), insbesondere zur Plasmakoagulation von biologischem Gewebe,
mit einer Fluidleitung (14), die wenigstens einen an eine Gasquelle (13) anschließbaren Kanal (14a) begrenzt, der zu einem distalen Ende (15) der Fluidleitung (14) führt und dort an einer Ausströmöffnung (16) endet,
mit einer in Nachbarschaft zu der Ausströmöffnung (16) angeordneten Elektrode (17), die über eine Leitung (18) an eine, elektrische Quelle (12) anschließbar ist,
**dadurch gekennzeichnet, dass** an der Elektrode (17) ein hydrophober Oberflächenbereich (21) ausgebildet ist.

2. Plasmasonde nach Anspruch 1, **dadurch gekennzeichnet, dass** die Fluidleitung (14) ein flexibler Schlauch aus elektrisch isolierendem Material ist, in dessen distalen Ende (15) ein elektrisch isolierendes, hitzefestes Endstück (30) gehalten ist, durch das sich der Kanal (14a) erstreckt.

3. Plasmasonde nach einem der vorstehenden Ansprüche, **dadurch gekennzeichnet, dass** die Elektrode (17) wenigstens teilweise in dem Endstück (30) angeordnet ist.

4. Plasmasonde nach einem der vorstehenden Ansprüche 2 oder 3, **dadurch gekennzeichnet, dass** ein hydrophober Oberflächenbereich (26) zusätzlich an dem Endstück (30) und/oder an dem Schlauch (14) ausgebildet ist.

5. Plasmasonde nach einem der vorstehenden Ansprüche, **dadurch gekennzeichnet, dass** die Elektrode (17) hohl ausgebildet ist und an ihrer Außenseite einen hydrophoben Oberflächenbereich (21) aufweist.

6. Plasmasonde nach einem der vorstehenden Ansprüche, **dadurch gekennzeichnet, dass** an oder in dem distalen Ende (15) ein Halter (19) angeordnet ist, der mit der Elektrode (17) verbunden ist und diese in der Fluidleitung (14) fixiert.

7. Plasmasonde nach Anspruch 6, **dadurch gekennzeichnet, dass** an dem Halter (19) zusätzlich ein hydrophober Oberflächenbereich (27, 28) ausgebildet ist.

8. Plasmasonde nach einem der vorstehenden Ansprüche, **dadurch gekennzeichnet, dass** der hydrophobe Oberflächenbereich (21) mit Wasser einen Benetzungswinkel (α) festlegt, der größer als 90° ist.

9. Plasmasonde nach einem der vorstehenden Ansprüche, **dadurch gekennzeichnet, dass** die Oberflächenenergie des hydrophoben Bereichs (21) kleiner als 20 mJ/m² ist.

10. Plasmasonde nach einem der vorstehenden Ansprüche, **dadurch gekennzeichnet, dass** der hydrophobe Bereich (21) durch hydrophobes Material gebildet ist.

11. Plasmasonde nach Anspruch 6, **dadurch gekennzeichnet, dass** der Halter (19) wenigstens zwei an der Elektrode (17) entlang führende Kanäle (33, 34) aufweist.

## Claims

1. A plasma probe (10), in particular for plasma coagulation of biological tissue,
having a fluid line (14) which delimits at least one channel (14a) that can be connected to a gas source (13), wherein the channel (14a) leads to a distal end (15) of the fluid line (14) and terminates there at an outlet opening (16),
having an electrode (17) arranged in proximity to the outlet opening (16) which can be connected to an electrical source (12) via a line (18),
**characterized in that** a hydrophobic surface area (21) is formed on the electrode (17).

2. The plasma probe according to claim 1, **characterized in that** the fluid line (14) is a flexible hose made of electrically insulating material, in the distal end (15) of which an electrically insulating, heat-resistant end piece (30) is held, through which the channel (14a) extends.

3. The plasma probe according to anyone of the preceding claims, **characterized in that** the electrode (17) is at least partially arranged inside the end piece (30).

4. The plasma probe according to anyone of the preceding claims 2 or **3, characterized in that** a hydrophobic surface area (26) is additionally formed on the end piece (30) and/or on the hose (14).

5. The plasma probe according to anyone of the preceding claims, **characterized in that** the electrode (17) is hollow and comprises a hydrophobic surface area (21) on its outer side.

6. The plasma probe according to anyone of the preceding claims, **characterized in that** a holder (19) is arranged on or in the distal end (15), wherein the holder (19) is connected to the electrode (17) and affixes it in the fluid line (14).

7. The plasma probe according to claim 6, **characterized in that** a hydrophobic surface area (27, 28) is additionally formed on the holder (19).

8. The plasma probe according to anyone of the preceding claims, **characterized in that** the hydrophobic surface area (21) defines a wetting angle of contact (α) with water which is greater than 90°.

9. The plasma probe according to anyone of the preceding claims, **characterized in that** the surface energy of the hydrophobic surface area (21) is less than 20 mJ/m².

10. The plasma probe according to anyone of the preceding claims, **characterized in that** the hydrophobic surface area (21) is formed by hydrophobic material.

11. The plasma probe according to claim 6, **characterized in that** the holder (19) comprises at least two channels (33, 34) extending along the electrode (17).

## Revendications

1. Sonde à plasma (10), destinée en particulier à la coagulation au plasma de tissus biologiques,
comprenant une conduite de fluide (14) qui délimite au moins un canal (14a) qui peut être raccordé à une source de gaz (13) et mène à une extrémité distale (15) de la conduite de fluide (14) où il se termine par un orifice de sortie (16),
comprenant une électrode (17) qui est disposée à proximité de l'orifice de sortie (16) et peut être raccordée par un câble (18) à une source électrique (12),
**caractérisée en ce qu'**une zone de surface hydrophobe (21) est réalisée sur l'électrode (17).

2. Sonde à plasma selon la revendication 1, **caractérisée en ce que** la conduite de fluide (14) est un tuyau souple constitué d'un matériau électriquement isolant, dans l'extrémité distale (15) du quel est tenue une pièce d'extrémité (30) électriquement isolante et résistant à la chaleur, à travers laquelle s'étend le canal (14a).

3. Sonde à plasma selon une des revendications précédentes, **caractérisée en ce que** l'électrode (17) est disposée au moins en partie dans la pièce d'extrémité (30).

4. Sonde à plasma selon une des revendications précédentes 2 ou 3, **caractérisée en ce qu'**une zone de surface hydrophobe (26) est réalisée en plus sur la pièce d'extrémité (30) et/ou sur le tuyau (14).

5. Sonde à plasma selon une des revendications précédentes, **caractérisée en ce que** l'électrode (17) est réalisée sous une forme creuse et présente une zone de surface hydrophobe (21) sur sa face externe.

6. Sonde à plasma selon une des revendications précédentes, **caractérisée en ce qu'**il est prévu sur ou dans l'extrémité distale (15), un support (19) qui est relié à l'électrode (17) et fixe celle-ci dans la conduite de fluide (14).

7. Sonde à plasma selon la revendication 6, **caractérisée en ce qu'**une zone de surface hydrophobe (27, 28) est en outre prévue sur le support (19).

8. Sonde à plasma selon une des revendications précédentes, **caractérisée en ce que** la zone de surface hydrophobe (21) définit avec l'eau un angle de mouillage (α) qui est supérieur à 90°.

9. Sonde à plasma selon une des revendications précédentes, **caractérisée en ce que** l'énergie superficielle de la zone hydrophobe (21) est inférieure à 20 mJ/m².

10. Sonde à plasma selon une des revendications précédentes, **caractérisée en ce que** la zone hydrophobe (21) est constituée d'un matériau hydrophobe.

11. Sonde à plasma selon la revendication 6, **caractérisée en ce que** le support (19) présente au moins deux canaux (33, 34) s'étendant le long de l'électrode (17).
